# EUROPEAN PATENT SPECIFICATION

(11) **EP 2 187 962 B1**
(45) Date of publication and mention of the grant of the patent: **11.03.2015**
(21) Application number: 08826035.1
(22) Date of filing: 24.04.2008
(51) Int. Cl.: A61K 39/205, C12N 5/00

(54) **ADAPTATION OF PITMAN MOORE STRAIN OF RABIES VIRUS TO PRIMARY CHICK EMBRYO FIBROBLAST CELL CULTURES**
ADAPTION EINES PITMAN MOORE-TOLLWUTVIRENSTAMMS AN PRIMÄRE HÜHNEREMBRYO-FIBROBLASTENZELLKULTUREN
ADAPTATION DE LA SOUCHE PITMAN-MOORE DE VIRUS RABIQUE À DES CULTURES CELLULAIRES DE FIBROBLASTES D'EMBRYON DE POULET PRIMAIRES

(30) Priority: 03.07.2007 IN MU12752007
(43) Date of publication of application: 26.05.2010
(60) Divisional application: 12180713.5
(73) Proprietor: Cadila Healthcare Limited, Ahmedabad 380 015, Gujarat (IN)
(72) Inventor: PATEL, Pradip, Maganlal, Gujarat (IN); PATEL, Pankaj, Ramanbhai, Gujarat (IN)
(74) Representative: Adams, Harvey Vaughan John
(86) International application number: PCT/IN2008/000262
(87) International publication number: WO 2009/004641

(56) References cited:
- US-A- 4 115 195
- US-A1- 2006 275 776
- WUNDERLI ET AL: "The rabies peripheral challenge test: More accurate determination of vaccine potency" VACCINE, vol. 24, no. 49-50, 9 November 2006 (2006-11-09), pages 7115-7123, XP005755630 ISSN: 0264-410X
- AMBROZAITIS ET AL: "Rabies post-exposure prophylaxis vaccination with purified chick embryo cell vaccine (PCECV) and purified vero cell rabies vaccine (PVRV) in a four-site intradermal schedule (4-0-2-0-1-1): An immunogenic, cost-effective and practical regimen" VACCINE, vol. 24, no. 19, 8 May 2006 (2006-05-08), pages 4116-4121, XP005421555 ISSN: 0264-410X

## Description

The present invention relates to the field of vaccine, in particular, the adaptation of Pitman Moore rabies virus strain (PM strain) into Purified Chick Embryo Fibroblast Cell culture for producing an improved and highly purified vaccine. Specifically the adapted PM rabies virus strain gives a high virus titer, which in turn gives high yields in terms of vaccine doses per egg and is also highly immunogenic.

### BACKGROUND AND PRIOR ART OF THE INVENTION

Rabies is a zoonotic viral disease which infects domestic and wild animals. Once symptoms of the disease develop, rabies is fatal to both animals and humans. However, when individuals are vaccinated with a rabies vaccine either prior to the virus exposure or after the exposure when combined with thorough cleaning of the wound with antiseptic and anti-rabies antibodies, the individuals are generally well protected. Human rabies vaccines are made from inactivated or attenuated rabies virus and have gone through successive improvements since the time of Pasteur. The first rabies vaccine was developed by Pasteur which was nerve tissue based and virus was inactivated by drying but this vaccine had a risk of activation of the virus and allergic reaction due to the presence of nerve tissue or myelin. Myelin free vaccines prepared from neonatal mouse brains were introduced by Fuenzalida et. al. (Vaccines: Fourth edition, chapter 37, Plotkin, Rupprecht and Koprowski*).*

Subsequently, Duck Embryo Vaccine (DEV) for rabies was developed. DEV for rabies was prepared from virus propagated in embryonated duck eggs. It was less immunogenic than the brain tissue vaccine. Fourteen to twenty three daily inoculations were recommended for DEV and sometime such high dosages also did not protect against rabies after severe exposure. *(*vaccines: Fourth edition, chapter 37, Page number 1018, Plotkin, Rupprecht and Koprowski*).* The other drawback associated with DEV was that it also had myelin based proteins, which caused side reactions so later on it was banned by the WHO. Thus there was a long need for highly immunogenic rabies vaccine that could be used safely and effectively at low doses, both for primary immunization and for treatment after exposure. These vaccines would also greatly reduce the number and severity of post vaccinal reactions.

Such a need was satisfied by the development of tissue/cell culture vaccines. The cell culture vaccine is not only safer compared to the former brain tissue vaccines by virtue of the absence of neuronal tissue but also is more efficacious. Several cell culture based vaccines have been developed in order to achieve high immunogenicity and safety like Purified Duck Embryo Vaccine (PDEV), 1^{st} generation vaccines like Human Diploid Cell Vaccine *(*Wiktor et al., 1964. J.Immuno1.93:353-366*) and 2^{nd} generation vaccines like Purified Chick Embryo Cell Vaccine (PCECV), Purified Vero Cell Rabies Vaccine, Rabies Vaccine Adsorbed (RVA), and Primary Hamster Kidney Cell Vaccine (PHKCV) etc. (Ref:* JIACM 2006; 7(1): 39-46*).*

The technical advancement leading to the development of the above vaccines included the adaptation of Pitman Moore strain of rabies virus to continuous cell lines such as Vero cells (e.g. Purified Vero Cell Rabies Vaccine - Abhayrab^{™} & Verorab^{™}) and MRC-5 human diploid cell culture line [e.g. Human Diploid Cell Vaccine (HDCV) - MIRV-HDC in India] [Ref: JIACM 2006; 7(1): 39-46] or in Duck Embryos in situ (e.g. Purified Duck Embryo Vaccine-PDEV-Lyssavac N) *(Ref:* Laboratory Techniques in Rabies; Fourth Edition, Edi. by F.X. Meslin, M. M. Kaplan & H. Koprowski, WHO Geneva-1996*).*

Both Vero and MRC-5 cell lines are continuous cell lines, and hence necessitate the testing of cellular residual DNA in the finished product (Ref. European Pharmacopoeia, 2004) which may be due to risk of either transmission of latent viruses & other agents. Moreover, the yields obtained with Vero cells are substantially low even when PM strain is used for preparing the vaccine. PDEV is a suspension vaccine and hence this vaccine does not qualify for Intra-dermal (ID) application. Moreover, the technology suffers from attaining low yields (1.8 - 2.2 doses/egg). The process time is also of 88 days which is too long. The commercially available PDEV vaccine uses a preservative thiomersal, which has been linked to possible Autism in young children (Ref: http://www.ncirs.usyd.edu.au/facts/f-thiomersal.html). Additionally, the process for production of PDEV is long, cumbersome and not preferred for large scale production because it gives low yield. HDCV is considered gold standards vaccine but it is highly expensive. Therefore, there is a need to provide an improved and highly immunogenic rabies vaccine which provides better yield as well as is less expensive using cell culture based technology.

US 4115195 (Rudolph Barth et al.*)* describes a process to manufacture rabies vaccine. It teaches that Chick Embryo Fibroblast cells, along with other cultures of cell strains can be used to make rabies vaccine with various viruses like viruses of strain VP 11, strain Pasteur, PM strain, or homogenized Chick-Embryo material containing viruses of strain Flury LEP (Low egg passage) or Flury HEP(High egg passage). The patent also specifically provides examples for use of rabies virus fixed strain VP 11, Flury HEP and Flury LEP to infect Chick Embryo Fibroblast cells. However, this document does not teach the adaptation of Pitman Moore strain (Wistar strain PM-HDCS, 1503-3M) either to Primary Duck Embryo Fibroblast Cells or to Primary Chick Embryo Fibroblast cells. Also, the media used in the present invention is a unique combination medium, which is not taught in US 4115195, and is exclusively designed for PM rabies virus to infect Primary Chick Embryo Fibroblast Cells.

The present inventors have surprisingly found that the Pitman Moore strain could be adapted to Primary chick fibroblast cell culture. Such adaptation has provides a method of preparing the rabies vaccine in large quantities, having excellent yield, with low throughput time and easily scalable. The vaccine produced will be suitable for Intra Dermal application in addition to intramuscular (IM) application, since the vaccine is not a suspension.

According to the present invention, the rabies vaccine prepared by adaptation of Pitman Moore to primary chick fibroblast cell culture is more advantageous than many other continuous cell lines based rabies vaccine and is more readily scalable to large scale commercial vaccine production. The vaccine produced by the present process has a very high yield, efficacy, safety as well as the process is much cost effective than many of the other processes known for preparation of rabies vaccine, preferably when the process is carried out using PET TC Roller bottles.

The present inventors have surprisingly found that Pitman Moore virus can be severely infected in Primary chick fibroblast cell culture with unique combination medium as described elsewhere, under suitable process conditions as hereinafter described in details, using PET TC Roller bottles. Such preferred embodiments provide vaccine with high yield, greater potency and immunogenicity which makes the vaccine comparatively cost effective.

### OBJECT OF THE INVENTION

The primary objective of the present invention is the adaptation of the Pitman Moore rabies virus strain to Chick Embryo Fibroblast cell culture in order to obtain a rabies vaccine.

In an embodiment of present invention is provided a process for the production of an immunogenic and highly Purified Chick Embryo Cell Vaccine^{PM} (henceforth called PCECV^{PM}) using Pitman Moore strain for active immunization against Rabies.

A further embodiment of the invention is to develop a high yielding unique process for obtaining the vaccine by a simpler process.

In a still further embodiment of the present invention is provided a suitable medium composition in order to achieve high infectivity of Pitman Moore strain rabies virus into Chick Embryo Fibroblast Cells.

### SUMMARY OF THE INVENTION

The present invention and embodiments thereof are set out in the appended claims.

The present invention provides a process for the adaptation of Pitman Moore rabies virus strain to Chick Embryo Fibroblast cell culture. The original Pitman Moore strain (Wistar strain PM-HDCS, 1503-3M) is first adapted to mice via one intracerebral passage and subsequently in duck eggs by repeated passages which the present inventors have further.adapted to the duck embryo fibroblast cells by successive passages, followed by adaptation to Primary chick embryo fibroblast cells by further serial passages using suitable medium and other suitable culture parameters to obtain the rabies vaccine.

Another aspect of the present invention provides a unique combination medium in order to achieve high and severe infectivity of Pitman Moore rabies virus strain into Purified Chick Embryo Fibroblast Cells.

In another aspect of the invention is provided an inactivated Purified Chick Embryo Cell Rabies Vaccine using PM rabies virus Strain having high purity and immunogenicity.

### BRIEF DESCRIPTION OF THE FIGURES

- Figure 1:: Shows rabies specific fluorescence of 03PM/Duck/S.Pas.07 in duck culture on day 5^{th}
- Figure 2:: Shows rabies specific fluorescence of 04PM/Chick/S.Pas.09 in Chick Embryo Culture (CEC) on day 5^{th}
- Figure 3:: Shows rabies specific fluorescence of 04PM/Chick/S.Pas.10 in Chick Embryo Culture (CEC) on day 3^{rd}
- Figure 4:: Shows rabies specific fluorescence of 04PM/Chick/S.Pas.11 in Chick Embryo Culture (CEC) on day 3^{rd}
- Figure 5:: Shows rabies specific fluorescence of 04PM/Chick/S.Pas.12 in Chick Embryo Culture (CEC) on day 3^{rd}
- Figure 6:: Comparison of Experimental Vaccine of the present invention with various Marketed rabies Vaccines.

### DETAILED DESCRIPTION OF THE INVENTION

The present invention relates to the adaptation of Pitman Moore rabies virus strain in primary cultures of Chick/Duck fibroblast cells. The eggs used are SPF quality chicken eggs. SPF chicken eggs and Pitman Moore rabies virus strain are approved substrate and approved virus strain by WHO for the manufacturing of the rabies vaccine for humans. In a preferred embodiment, the PM rabies virus strain is adapted to chick fibroblast cells in roller cultures using PET TC roller bottles which gives higher yield compared to several other technologies.

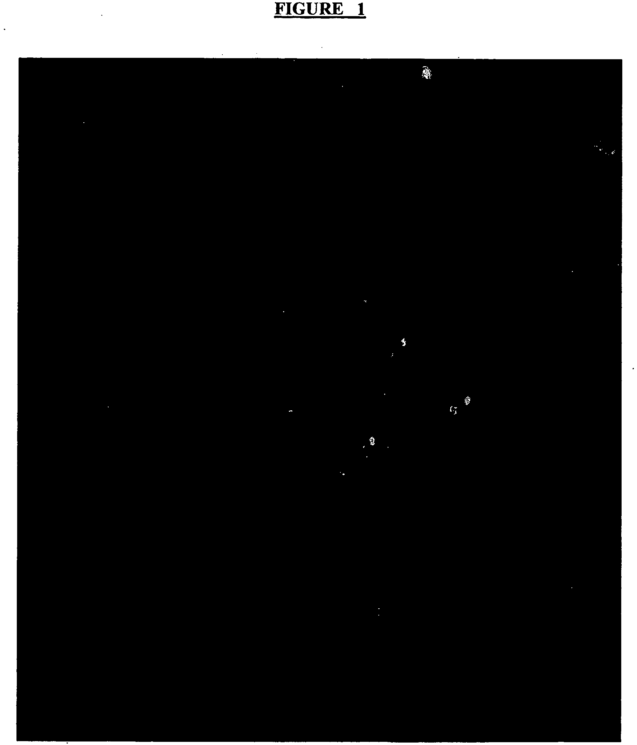

The original Pitman Moore strain (Wistar strain PM-HDCS, 1503-3M) is first adapted to mice via one intracerebral passage and subsequently in duck eggs by repeated passages. Subsequently, the present inventors attempted to develop an adaptation process of PM rabies virus strain to Primary Duck Embryo Fibroblast Cells, only some cells were found to be infected (Figure 1). Over several passages, the infectivity was not increased to the satisfactory extent.

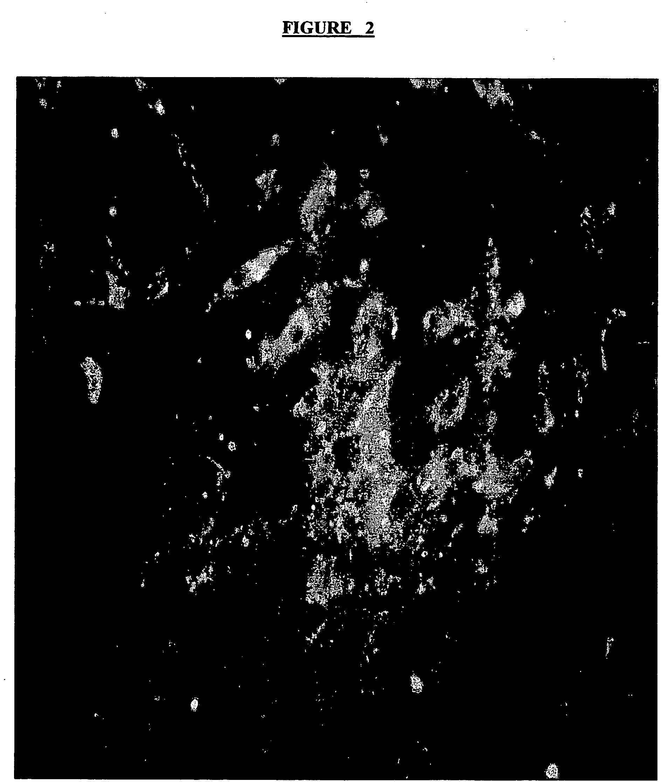

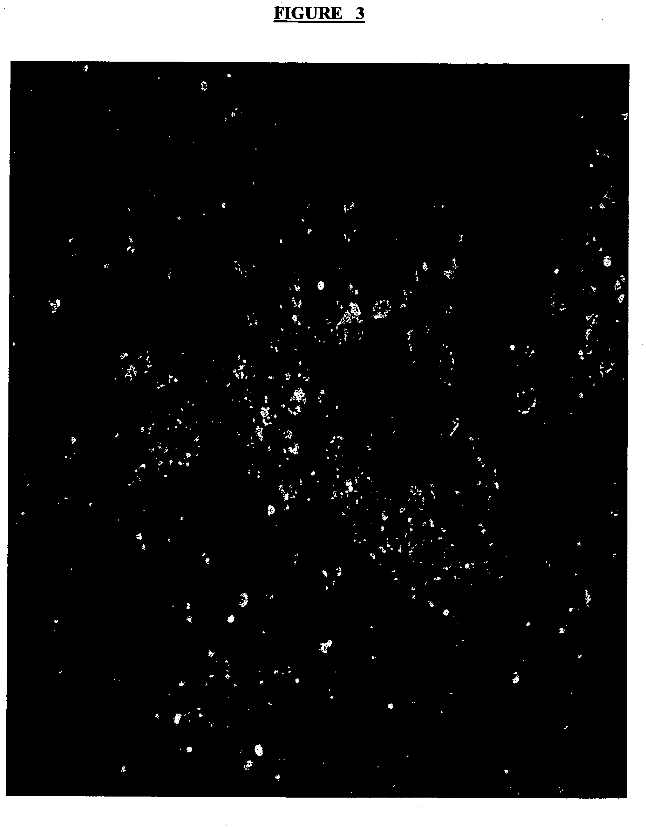

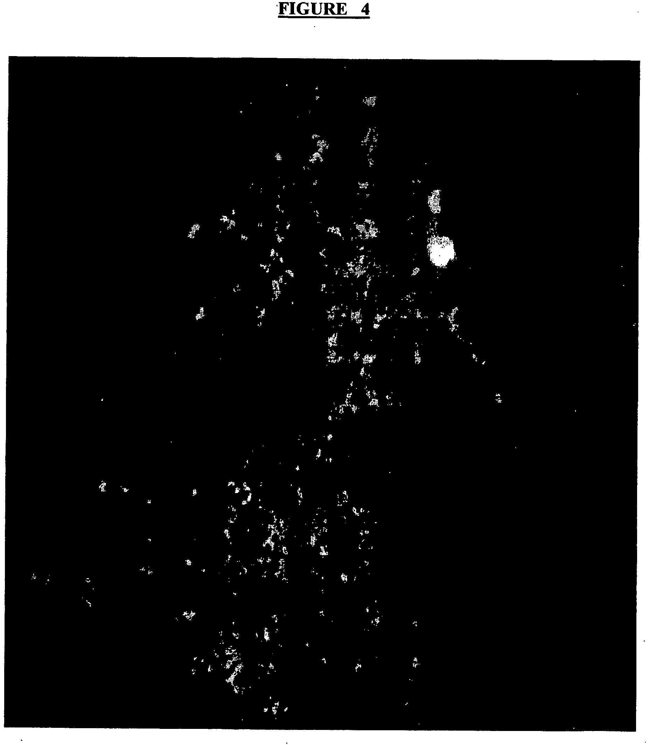

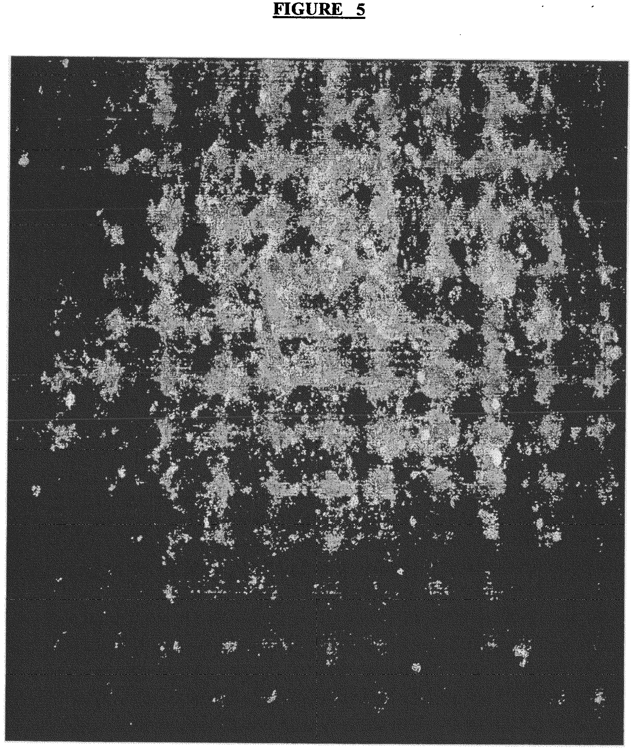

It is known, for e.g. in US 4115195, that a sufficiently high virus titer is a pre-requisite for an effective rabies vaccine Hence, the inventors changed their focus to give passages of the virus into Chick Embryo Fibroblast cell culture. After several experiments for infectivity and adaptation, in chick embryo cells, successful results of virus titration were obtained (Figure 2 to 5).

The original "Wistar strain PM-HDCS, 1503-3M" adapted to duck embryo is herein referred to as "Master Seed DE 42/74 Pas. 12 12.11.1974" which is a master seed for production of PDEV vaccine. This "Master Seed DE 42/74 Pas. 12 12.11.1974" rabies virus is adapted and propagated initially in duck embryo cells then to Primary Chick Embryo Fibroblast Cell Cultures to produce cell culture PCEC Vaccine (PCECV^{PM}) for rabies according to the present invention.

Initially, the inventors attempted to adapt the "Master Seed DE 42/74 Pas. 12 12.11.1974" virus to Primary Duck Embryo Fibroblast Cells by repeated passages. But, since Primary Duck Embryo Fibroblast cells are not a 'natural' host for the PM rabies virus, the inventors could not get any infection in the initial experiments, and further optimizations only resulted in very little infection in subsequent experiments. Hence, the process was significantly modified and the passaging in Primary Duck Embryo Fibroblast cells was shifted to passaging in Primary Chick Embryo Fibroblast cells under suitable medium and culture parameters. Such passaging of PM strain of rabies virus into Primary Chick Embryo Fibroblast cells is a unique and not a routine substitution which is neither reported in the art nor can be extrapolated by a person skilled in the art as routine, since these cells are also not a 'natural' host to the PM strain of Rabies virus.

In the present invention, the "Master Seed virus DE 42/74 Pas.12 12.11.1974" was adapted to Primary Duck Embryo Fibroblast Cells, and then to Primary Chick Embryo Fibroblast Cells by repeated passages as described below:

Wistar strain PM-HDCS, 1503-3M
One pas.( mouse brain )
11 passages (duck Eggs)
DE/PM Pas 11; 29.05.1972
1 Pas. (duck eggs )

PM virus (DE 42/74 Pas. 12 12.11.1974)
Infection into Primary Duck Embryo
Fibroblast Cells
02 PM/Duck/S.Pas.01 ; 14.04.02
3-6 Passage given for infection, amplification
& adaptation
03 PM/Duck/S Pas.07; 18.12.03
Infection into Primary Chick Embryo
Fibroblast Cells
04 PM/Chick / S. Pas. 08; 27.04.04
Fully adapted by atleast 3 passages
04 PM/Chick/S Pas.11; 04.08.04 (Master Seed)
1-5 Passages
04 PM/Chick/S Pas.12; 25.12.04
(Working Seed)

The nomenclature for the various strains obtained during passaging are in the following general format:
*"year of preparation (e.g. '04' for 2004)lPitman Moore strain(PM)*/*substrate in which passaging took place (duck or chick)*/*serial passage number*/*date of culture"*

The adapted Pitman Moore strain which is used as a master seed or working seed is obtained by infecting PM virus (DE 42/74 Pas. 12 12.11.1974) to Primary Duck Embryo Fibroblast Cells and 7 passages were performed at temperature at 33-36 °C in order to adapt the strain in the Primary Duck Embryo Fibroblast Cells and obtained "03 PM/Duck/S Pas.07 18.12.03". The "03 PM/Duck/S Pas.07 18.12.03" strain was further infected in to Primary Chick Embryo Fibroblast Cells by at least 4 passages to obtain the master seed "04 PM/Chick/S Pas.1104.08.04". One can give 1-5 passages from Master seed to prepare the working seed for commercial vaccine production. One Example of working seed lot prepared according to the above process was named as "04 PM/Chick/S Pas.12 25.12.04".

The virus obtained from the Master seed "04 PM/Chick/S Pas. 11 04.08.04" has a peak titer of at least 10^{6.5} TCID_{50%} per ml.
The virus titer was in increasing log at successive passages in chick embryo cell, suggesting that the virus has stable phenotypic character.

At every stage of passage, infectivity was checked by staining culture with Ab tagged- FITC conjugate using fluorescence microscopy. After getting optimum and reproducible conditions for infectivity and culture, the results were confirmed primarily by Fluorescence Microscopy and then by several in-vitro as well as in-vivo tests as described in various Pharmacopoeia.

The adaptation of the virus is preferably carried out using PET TC roller bottles or multi layered TC flasks.

For proper adaptation, several medium and their combinations of ingredients were employed in described below with the help of Example 1. The present invention is further illustrated by the following non-limiting examples which represent one of the preferred mode of carrying out the invention. It will be appreciated that several non-inventive modifications, which are within the scope of a person skilled in the art are possible which are considered to be within the scope of the present invention.

### Example 1: Selection of medium for virus adaptation and process optimization

Following media were chosen for virus adaptation and process parameters optimization.
- Production Culture Medium 1(PCM 1)
- Production Culture Medium 2 (PCM 2)
- Production Culture Medium (PCM)

The above mentioned medium comprises below ingredients:

| MEDIUM COMPOSITION | PCM 1 | PCM 2 | PCM |
|---|---|---|---|
| | Mg/L | Mg/L | Mg/L |
| **INORGANIC SALTS** | | | |
| Calcium chloride anhydrous | 200 | 200 | 175-225 |
| Ferric (III)-Nitrate. 9H2O | 0.1 | | 0.02-0.07 |
| Magnesium sulfate anhydrous | 97.7 | 97.7 | 72.7-122.7 |
| Potassium chloride | 400 | 400 | 375-425 |
| Sodium chloride | 6400 | - | 3175-3225 |
| Sodium Dihydrogen phosphate. H2O | 125 | 140 | 107.5-157.5 |
| Sodium hydrogen Carbonate | 3700 | 2200 | 2925-2975 |

| **VITAMINS** | | | |
|---|---|---|---|
| D-calcium pantothenate | 4 | 1 | 0.5-5.0 |
| Choline chloride | 4 | 1 | 0.5-5.0 |
| Folic acid | 4 | 1 | 0.5-5.0 |
| Myo-Inositol | 7.2 | 2 | 0.6-8.6 |
| Nicotinamide | 4 | 1 | 0.5-5.0 |
| Pyridoxal HCl | 4 | 1 | 0.5-5.0 |
| Riboflavin | 0.4 | 0.1 | 0.01-0.5 |
| Thiamine HCl | 4 | 1 | 0.5-5.0 |

| **AMINO ACIDS** | | | |
|---|---|---|---|
| L-arginine HCl | 84 | 126 | 80-130 |
| L-cystine | 48 | 24 | 5-30 |
| L-glutamine | 584 | 292 | 413-463 |
| Glycine | 30 | - | 20-40 |
| L-histidine HCl. H2O | 42 | 42 | 17-67 |
| L-isoleucine | 105 | 52 | 53.5-103.5 |
| L-leucine | 105 | 52 | 53.5-103.5 |
| L-lysine HCl | 146 | 72.5 | 84.25-134.25 |
| L-methionine | 30 | 15 | 2.5-42.5 |
| L-phenylalanine | 66 | 32 | 24-74 |
| L-serine | 42 | - | 16-26 |
| L-threonine | 95 | 48 | 46.5-96.5 |
| L-tryptophan | 16 | 10 | 10-30 |
| L-tyrosine | 72 | 36 | 29-79 |
| L-valine | 94 | 46 | 45-95 |
| OTHER | | | |
| D-Glucose anhydrous | 4500 | 1000 | 2725-2775 |
| Phenol red | 15 | 11 | 10-30 |
| Sodium pyruvate | 110 | - | 30-80 |

The above media were further supplemented with human serum albumin, hydrolyzed gelatin, sodium bicarbonate and antibiotics solution at suitable concentrations.

Following experiments were carried out using different mediums and their effectiveness were compared for formation of cell monolayer and degree of virus infection.

9-11 days old embryos were taken and trypsinized 10-10-20 minutes steps. The cells were centrifuged to remove trypsin and re-suspended equally in PCM 1, PCM 2 and PCM. Cell counts were adjusted to approx. 1.7 x 10⁶ cells per ml in all the three medium in different experiments. The virus "03 PM / Duck / S. Pas. 07" virus at 1:1200 dilution were used in all experiments. Adsorption time was 90 minutes for every experiments. The infected cells were seeded in Greiner TC flasks and PET Roller bottles. Representative infected cells from the TC flask/roller bottles were also seeded in 24 well TC plates. The TC flask & Roller bottles were incubated at 34 ⁰C ± ) 1 ⁰C for 5 days. The 24 well plate was incubated in 3 % CO₂ environment at 34 ⁰C ± 1 ⁰C and was stained with FITC conjugate on 3^{rd} day to check degree of virus infection which was rated by Rabies specific fluorescence as good (+), very good (++), excellent (+++) or extraordinary (++++).

On day 4^{th} or 5^{th}, cell supernatant was harvested from each set of experiment as first harvest and replenished with fresh respective culture medium. After further incubation, On day 2^{nd}, 3^{rd} or 4^{th} from the first harvest, second harvest were taken from each set. The first harvest and second harvest were tested for virus titration and sterility.
It was found that:
- In 24 well plate, cell seeded PCM 1 and PCM 2 showed poor to good infection while cell seeded in PCM showed excellent to extraordinary infection, evaluated by rabies specific immunofluorescence.
- TC flask / roller bottles seeded with PCM had shown good titers compared to PCM 1 and PCM 2 alone. Titer values, 10 ^{5.6}, 10 ^{5.8} and 10 ^{7.7} TCID_{50%} are found in PCM 1, PCM 2 and " PCM " respectively.
From the above results in various experiments, the medium PCM was chosen for adaptation in Primary chicken embryo fibroblast cells and subsequent vaccine preparation which was further optimized by various process parameters which are within the scope of persons skilled in the art and by further selection of suitable TC roller bottle.

### General cell culture procedure used for preparing the Master seed, Workin seed and rabies vaccine production:

The chicken eggs were incubated for 8 -11 days at a temperature 35° C - 37° C with 70 - 90 % RH. After incubation, the eggs were candled for live and healthy embryos. After candling, the embryos were removed from each egg under aseptic conditions then the heads and bodies were separated by pulling method. The heads were then immediately discarded while the body part of embryos were pooled in a bottle, washed three to four times with sterile PBS and trypsinized with trypsin prewarmed at 35 ± 2 ⁰C for 10-40 minutes.

Trypsinized cell suspension was filtered through a nylon muslin cloth followed by centrifugation at 1000 to 1500 rpm for 10 to 15 min in refrigerated centrifuge. The cell pellet is suspended in fresh growth medium, mixed well and again this cell suspension was centrifuged in the same manner. The cell pellet was suspended in fresh growth medium and mixed thoroughly. The cell suspension was transferred into a sterile glass bottle containing above said culture medium and stirred for 5-10 minutes at 35 ± 2 ⁰C. The final cell counts were adjusted in the range of 1.4 - 2.2 X 10⁶/ml using culture medium in 20 L glass bottle.

The cell suspension was inoculated with pre-determined optimum master or working seed virus dilution and incubated under slow stirring at 35 ± 2 ⁰C for 90-120 minutes for adsorption of virus to cells. At the end of adsorption, infected virus suspension were distributed either in PET TC roller bottles ( 300 ± 50 ml per TC Roller bottle ) or in multilayer TC flask such as cell factories or cell stacks ( 3000 ± 500 ml ) or combination of both. They were then incubated at 34.5 ± 0.5 °C for 4 to 6 days. On day 4^{th} or 5^{th}, cell supernatant was harvested as first harvest and replenished with fresh culture medium. After further incubation, second was taken on day 7^{th} or 8^{th}. Third harvest is optional. Various virus harvests were stored at 2⁰-8 ⁰C. Multiple harvest make the invention cost effective by getting more harvest with good titers

The pooled virus harvest were purified and concentrated by ultracentrifugation in a sucrose density gradient zonal centrifuge at 35000 rpm. Banding of the rabies virus takes place best between a sucrose concentration of approximately 35 - 40% and the sucrose gradient / bands having active live rabies Virus were collected as product fractions. The product fraction virus concentrates from various pooled harvests were stored below - 60° C until results of various test like In-vitro Ag assay, sterility test and Bacterial endotoxin tests were clear.

The present invention also provides a method of inactivating PM virus to destroy its infectivity while preserving its antigenicity. The concentrates were thawed at 37 ⁰C and cooled at 4 ± 2 ⁰C. The concentrates were diluted by vaccine stabilizer to achieve appropriate antigen content of 8.5 IU/ml and sample were withdrawn for In-vitro Ag assay like ABT, ELISA and SRD test. The pH of the blended bulk was measured and adjusted to 8.0 ±1 with 10% w/v pre-sterilized NaOH solution. Then effective quantity of Betapropiolactone diluted 1:100 in PBS was slowly added into the blended bulk so as to reach a final concentration of I: 4000 (0.025%) and the content of vessel was transferred into another pre-sterilized inactivation vessel. The incubation with Betapropiolactone was performed at 2⁰ to 6 ⁰C under constant stirring. At least 48 hours were required to fully inactivate viral infectivity without losing viral antigenicity. The inactivation process of PM virus at 2 ⁰C 6 ⁰C was preferably chosen for simplicity in large scale culture.

Finally the inactivated bulk was Stored at 5⁰ ± 3⁰C until further processing under continuous stirring. The inactivated bulk was diluted with suitable stabilizer solution to adjust antigenic value of atleast 5 IU/ml. During the blending process, the blended bulk was maintained at 5⁰ ± 3 ⁰C under stirring. The blended vaccine was stirred for 30 minutes prior to filling. Lyophilisation was done by standard methods known in the art. Vaccine stabilizer used contains sucrose, hydrolyzed gelatine, human albumin and sodium salts. The vaccine is prepared as a freeze-dried formulation to be reconstituted with sterile water for injection.

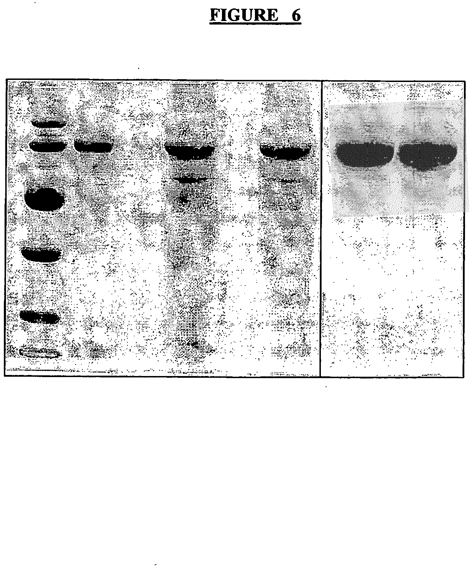

The efficacy and immunogenicity of vaccine prepared using chick fibroblast cells is same as International Reference Vaccine ( WHO 5^{th} IRM ) which has been evaluated by various animal safety, efficacy and seroconversion studied. Further, the end product purity is verified and compared by SDS page which is similar to other marketed products (Figure 6). The product is also very stable when stored at 2⁰ to 8 ⁰C .It also retains potency and other critical parameters when exposure at accelerated and stressed temperatures as per ICH guidelines for stability of Biotechnological and biological materials.

### Example 2: Adaptation of Pitman Moore Rabies Virus Strain to Primary Duck Embryo Fibroblast cells and successive adaptation of the virus obtained therein to Primary Chick Embryo fibroblast Cells.

(A) The original Pitman Moore rabies virus strain (Wistar strain PM-HDCS, 1503-3M) was first adapted by 1 cerebral passage in Swiss Albino Mice followed by 12 passages in embryonated Duck eggs, and the virus obtained was named as DE 42/74 Pas. 12 12.11.1974. The present invention uses this virus for further adaptation to primary duck embryo fibroblast cells and subsequently chick embryo fibroblast cells.

The experiment was carried out by using 11-12 days old SPF duck eggs. The cell count was set at 1.8 X 10⁶ cell/ml as per techniques known, cell suspension was prepared by using Production Culture Medium 1 (PCM 1, described above) with supplement of 1.5% human albumin. 5ml frozen aliquot of above named virus "DE 42/74 Pas. 12 12.11.1974" was thawed at 37 ⁰C and diluted in cell culture stabilizer, and was used for infecting the duck embryo cell suspension. The infected cell suspension was filled into TC PET roller bottles and incubated at 34 ⁰C. On day five after the infection, the harvest was collected from the roller bottles and tested for virus titer in mice and for absence of contaminants like bacteria and fungi. These harvested virus was named as "02 PM / DUCK / S Pas.01"

Like wise six such passages were further done in duck embryo cell culture and after each passage, harvest of approximately 7.0 liters. After each passage the harvest was collected and tested for virus titer in mice and for absence of contaminants like bacteria and fungi. The harvest from each passage was further distributed in 5ml aliquots. They were named as follows.

| Culture date. | No of eggs | Approximate Vol. of harvest | Ex. 1: Nomenclature |
|---|---|---|---|
| 14.04.02 | 180 | 07 ltrs | 02 PM / Duck / S. Pas. 01 |
| 27.07.02 | 180 | 07 ltrs | 02 PM / Duck / S. Pas. 02 |
| 30.11.02 | 180 | 07 ltrs | 02 PM / Duck / S. Pas. 03 |
| 11.01.03 | 180 | 07 ltrs | 03 PM / Duck / S. Pas. 04 |
| 04.10.03 | 180 | 07 ltrs | 03 PM / Duck / S. Pas. 05 |
| 17.11.03 | 180 | 07 ltrs | 03 PM / Duck / S. Pas. 06 |
| 18.12.03 | 180 | 07 ltrs | 03 PM / Duck / S. Pas. 07 |

During the above experiments, after initial passage, only few cells were found infected and showed rabies specific fluorescence. The infectivity of virus gradually and significantly increased from the fourth passage onwards. This can be shown as in the below chart:

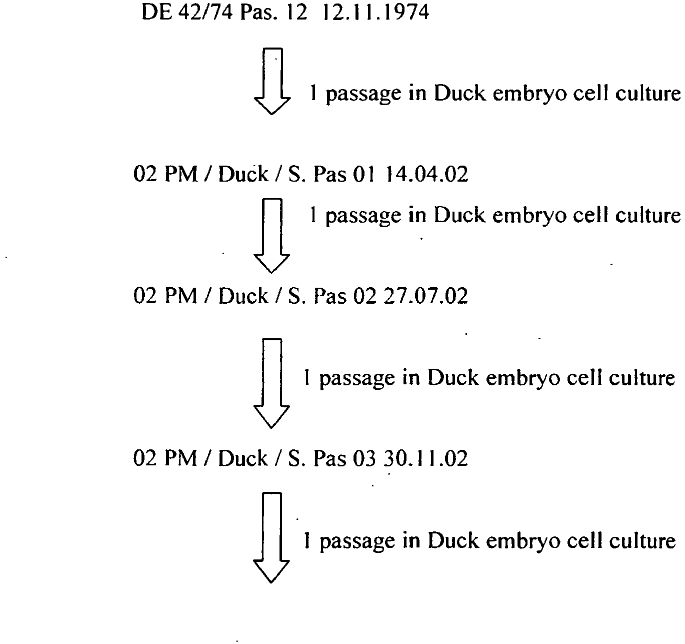
DE 42/74 Pas. 12 12.11.1974
1 passage in Duck embryo cell culture
02 PM / Duck / S. Pas 01 14.04.02
1 passage in Duck embryo cell culture
02 PM / Duck / S. Pas 02 27.07.02
1 passage in Duck embryo cell culture
02 PM / Duck / S. Pas 03 30.11.02
1 passage in Duck embryo cell culture

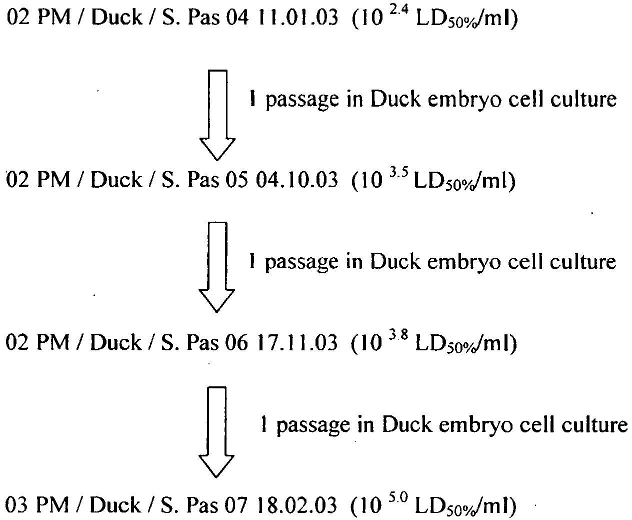
02 PM / Duck / S. Pas 04 11.01.03 (10 2.4 LD50%/ml)
1 passage in Duck embryo cell culture
02 PM / Duck / S. Pas 05 04.10.03 (10 3.5 LD50%/ml)
1 passage in Duck embryo cell culture
02 PM / Duck / S. Pas 06 17.11.03 (10 3.8 LD50%/ml)
1 passage in Duck embryo cell culture
03 PM / Duck / S. Pas 07 18.02.03 (10 5.0 LD50%/ml)

After seventh passage infectivity was almost constant. Several alternatives were tried with very little success. Surprisingly, when the virus "03 PM / Duck / S. Pas 07 18.02.03" was infected into chick embryo fibroblast cells, the infectivity increased significantly. Based on this findings, the virus strain was further passaged in chick embryo fibroblast cells in order to achieve required infectivity and virus titer as follows.
(B) Approximately 9-11 days old (180 embryos) fertilized SPF chicken eggs was used for the process and the head part was separated from the body part, the body part only was processed, while the head part was discarded. The pooled body parts were given repeated phosphate buffered saline wash and subsequently the embryos were trypsinized using trypsin solution. Cell suspension was prepared in PCM, the cell count was set at 1.6 x 10 ⁶ cells/ml. The 5ml frozen aliquot of "03 PM / Duck / S. Pas. 07" was thawed at 37 ⁰C and diluted in stabilizer. This was used for infecting the chick embryo cell suspension. This infected cell suspension was incubated at 37 ⁰C for 1.5 hrs with slow stirring. The infected cells were distributed into roller bottle as well as in multilayered TC flasks, and incubated at 34 ⁰C. On day five of the infection, the harvest was collected from the roller bottles and multilayered TC flasks, approximately 7 ltrs of harvest was collected and was distributed in suitable aliquots and from these aliquots few aliquots of 5 ml were used for testing virus titer in mice and for absence of contaminants like bacteria and fungi.

These virus harvest was given nomenclature as" 04 PM / Chick / S. pas .08"

| Culture date | No of eggs | approximate Vol. of harvest | Nomenclature |
|---|---|---|---|
| 27.04.04 | 180 | 07 ltrs | 04 PM / Chick / S. Pas. 08 |
| 11.05.04 | 180 | 07 ltrs | 04 PM / Chick / S. Pas. 09 |
| 16.07.04 | 180 | 07 ltrs | 04 PM / Chick / S. Pas. 10 |
| 04.08.04 | 180 | 07 ltrs | 04 PM / Chick / S. Pas. 11 |
| 25.12.04 | 180 | 07 ltrs | 04 PM / Chick / S. Pas. 12 |

Subsequently, 3 further successive passages of the virus were given in chicken fibroblast cells, aliquots from each harvest were collected and distributed in 5 ml aliquots and were given the nomenclature as mentioned above. All the passage were tested for virus titer and absence of bacteria and fungi.
Based on data obtained, the virus obtained after the 4^{th} passage named "04 PM / Chick / S Pas. 11 04.08.04" has 10 ^{7.7} LD_{50%} virus titer per ml which was herein considered as Master Seed Virus. This virus was given 1 further passage to produce the Working Seed Virus "04 PM / Chick / S Pas. 12 25.12.04"

### Example 3: Preparation of vaccine from the working seed

The seed Virus "04 PM / Chick / S Pas. 12 25.12.04" was infected in chicken fibroblast cell by a process similar to that described in step 1(b) above, and first harvest was obtained on or after 4 days and the second harvest was obtained after 2-3 days of the first harvest. The pooled virus harvest were purified and concentrated by ultracentrifugation by a sucrose density gradient zonal centrifuge at 35000 rpm. Banding of the rabies virus was carried out at approx. 35 - 40% of sucrose concentration and the active live rabies Virus were collected as product fractions. The virus concentrates from various pooled harvests were stored below - 60⁰ C until results of various test like in-vitro Ag assay, sterility test and bacterial endotoxin tests were clear.

The concentrates were thawed at 37 ⁰C and cooled at 4 ± 2⁰C. The concentrates were diluted by vaccine stabilizer to achieve appropriate antigen content of 8.5 IU/ml and sample were withdrawn for In-vitro Ag assay like ABT, ELISA and SRD test. The pH of the blended bulk was measured and adjusted to 8.0±1 with 10% w/v pre-sterilized NaOH solution. The blended bulk was then inactivated by addition of suitable concentration of Betapropiolactone, by known techniques.

Finally the inactivated bulk was stored at 5⁰ ± 3⁰C under continuous stirring and was diluted with suitable stabilizer solution to adjust antigenic value at ≥ 5 IU/ml and blended bulk was maintained at 5⁰ ± 3⁰C under stirring for 30 minutes. The blended bulk was filled in vials and using suitable stabilizer composition comprising sucrose, hydrolyzed gelatine, human albumin and sodium salts. The vaccine is made available as a freeze-dried formulation to be reconstituted with sterile water for injection prior to use.
The immunogenicity and potency of the vaccine were tested as follows:

### Example 4: NIH Potency test in Mice

The NIH Potency test in mice is carried out as per Laboratory Techniques in Rabies, 4th ED. WHO'1996 and Indian Pharmacopoeia.

The potency of rabies vaccine is determined by comparing the dose of the experimental vaccine necessary to protect mice against a lethal intra-cerebral dose of rabies virus with the corresponding dose of reference vaccine (WHO 5^{th} IRM, 16.0 IU per Ampoule) necessary to give the same protection.

Swiss Albino Mice weighing approximately 12-15 grams were used.

| No. of Mice Required | |
|---|---|
| Standard Vaccine | 64 ( 16 x 4 Dilutions ) |
| Test Vaccine | 64 ( 16 x 4 Dilutions ) |
| Virus Titration | ( 10 x 4 Dilutions) |

One dose of International Reference vaccine was reconstituted in 2.0 ml of water for injection, and 0.5 ml of it was mixed thoroughly with 12 ml of Phosphate Buffer Saline (PBS) to make 25 fold dilution.

One dose from at-least each shelves of freeze dried lots of the experimental vaccine of the invention were reconstituted separately in 1.0 ml of water for injection, and then pooled together.Take out 0.5 ml which was mixed thoroughly with 12 ml of PBS thereby making a 25 fold dilution.

Subsequently, three five fold dilutions of both International Standard and Experimental vaccines were prepared in PBS (pH 7.4). The range of dilutions were so selected that the middle dilution contained enough vaccine to protect 50 % of total number of mice taken for the study, with the challenge dose of virus of 10-50 LD₅₀.The dilution range of 1:25, 1:125, 1:625, 1:3125 worked well for both, standard as well as experimental vaccine.

During first immunization sixteen mice (8 Males + 8 Females) were injected with 0.5 ml of each dilution, by intraperitoneal route and second immunization was done after seven days from the day of first immunization, repeating the steps as that of first vaccination.

### Virus Challenge (Infection) to the Immunized Mice

Fourteen days after I^{st} immunization, all mice were infected with Brain Suspension of Challenge Virus Standard (CVS); dose of which adjusted between 10 - 50 LD₅₀.

### Observation of Experimental Animals

Infected mice are observed for 14 days. Mice deaths are noted after fifth day of infection.

### Calculation of Potency

The potency was calculated by REED and MUENCH method. It was found that:
- The ED₅₀ values of the Test as well as Standard Vaccines were between the highest and the lowest dose administered to the experimental animals.
- The titration of the challenge suspension showed that 0.03 ml. of it contained between 10 to 50 LD₅₀ and all the animals should die which received the suspension of this strength.

The confidence limits of the level of significance (p = 0.95) were not less than 25% and not more than 400% of the calculated potency.

The final result is tabulated below:

| Lot No. | Test | Potency (IU/dose) | Confidence Limit (95%) | Challenge Virus Dose | Mean Potency (IU/dose) |
|---|---|---|---|---|---|
| 1 | I | 5.42 | 2.667 - 10.928 | 26.30 | 5.43 |
| | II | 5.50 | 2.541 - 11.928 | 26.92 | |

From the above experiment, it can be concluded that the mean potency of the experimental vaccine is 5.43 IU/dose. The 95% confidence limit of each test is between 25-400% of the calculated potency. The challenge virus dose (CVD) used in each test was between 10-50 LD₅₀. ED₅₀ of standard and test vaccine in each test was between the highest and lowest dilution. Hence it can be concluded that the vaccine is immunoprotected and NIH potency value is well above the minimum requirement of 2.5 IU/dose.

### Example 5: Seroconversion and Challenge study in Rabbits

From the positive results obtained in mice in terms of seroconversion/challenge study, a combined study was designed to ascertain seroconversion and protection in rabbits. New Zealand White Rabbits having weight within 1.5 - 2.5 Kgs were used, 5 males & 5 females rabbits were taken per group,

WHO 5^{th} IRM, reconstituted in 2 ml of Sterile Water for Injections to get 8 IU/ml was used as Reference vaccine. The Experimental vaccine of the present invention was reconstituted in 1 ml of Sterile Water for Injection.

All the rabbits in each group were injected intra-muscularly with 0.5 ml of respective vaccines on day 0 and on day 7 and were observed for 14 days. On day 14, 2 ml of blood was collected aseptically from Marginal Ear vein of each rabbit and blood was allowed to clot by incubation at 37 °C for 1 hour. The blood samples were centrifuged at 1,500 rpm / 10 mins for serum separation and serum was collected aseptically in pre-sterilized tubes/cryovials. The samples were heat-inactivated at 56°C for 30 minutes and stored below -60 °C for further titer determination.

The antibody titer in each sample was determined by RFFIT & MNT. Subsequently, all rabbits were challenged intra-cerebrally with 30MLD₅₀/0.3ml of Challenge Virus Standard (CVS-27) under Intravenous anaesthesia (Thiopentone sodium). The rabbits were observed for 14 days for Rabies specific symptoms and mortality. The mortality upto day 5^{th} was considered non-specific death.

### OBSERVATION:

It was observed that none of the rabbit in any group exhibited Rabies specific symptoms or died. The test vaccine was similar in immunogenicity with respect to reference vaccine as can be inferred from the table.

### Sero Conversion in Rabbit

**Group I: Reference Rabies Vaccine (WHO 5^{th} IRM)**

| Rabbit No. | Sex | Antibody titer (IU/ml) | |
|---|---|---|---|
| | | RFFIT | MNT |
| 1 | Male | 4.65 | 9.6 |
| 2 | Male | 7.39 | 11.5 |
| 3 | Male | 8.10 | 10.0 |
| 4 | Male | 6.92 | 11.5 |
| 5 | Male | 8.10 | 8.91 |
| 6 | Female | 5.86 | 11.0 |
| 7 | Female | 11.73 | 10.0 |
| 8 | Female | 12.53 | 8.91 |
| 9 | Female | 8.10 | 6.0 |
| 10 | Female | 11.73 | 13.2 |
| Mean | - | 8.51 | 10.06 |
| S.D. | - | 2.65 | 1.95 |

**Group II : Experimental Test Vaccine (PCEC rabies vaccine)**

| Rabbit No. | Sex | Antibody titer (IU/ml) | |
|---|---|---|---|
| | | RFFIT | MNT |
| 1 | Male | 11.73 | 9.12 |
| 2 | Male | 8.10 | 9.14 |
| 3 | Male | 5.86 | 8.91 |
| 4 | Male | 6.92 | 11.48 |
| 5 | Male | 7.39 | 12.88 |
| 6 | Female | 8.10 | 13.80 |
| 7 | Female | 4.65 | 13.20 |
| 8 | Female | 6.92 | 11.48 |
| 9 | Female | 12.53 | 12.0 |
| 10 | Female | 8.10 | 6.70 |
| Mean | - | 8.2 | 10.87 |
| S.D. | - | 3.2 | 2.29 |

### INFERENCE:

From the antibody titer obtained for experimental test vaccine (Mean: 8.2 & 10.87 by RFFIT & MNT respectively) as well as reference vaccine (Mean: 8.51 & 10.06 by RFFIT & MNT respectively), it was concluded that the Experimental vaccine of the present invention is equally immunogenic as the reference vaccine.

Therefore, one can conclude from the results obtained after challenge, that the PCEC vaccine (Experimental vaccine) prepared according to the process of the present invention is equally protective as the reference standard in terms of immunogenicity and potency.

### Example 6: Comparision of Experimental Vaccine of the present invention with various Marketed Rabies Vaccines

The SDS page analysis was carried out to check and compare the protein banding of the Experimental rabies vaccine with those of other marketed products using known markers.

From the study it was observed (Figure 6) that the virus concentrate of the present invention when formulated like other marketed rabies vaccines showed identical protein bands.
In the Figure 6,
- Lane 1: : Low molecular weight marker;
- Lane 2: : API of the present invention;
- Lane 3: : API of the present invention formulated like other marketed PCEC rabies vaccine;
- Lane 4: : Marketed PCEC rabies vaccine;
- Lane 5: : Vero cell line based marketed rabies vaccine;
- Lane 6: : API of the present invention formulated like other marketed Vero rabies vaccine.

## Claims

1. A Primary chick embryo fibroblast cell comprising a Pitman Moore strain of rabies virus adapted to the Primary chick embryo fibroblast cell according to a process comprising the steps of:
a) adapting the Pitman Moore strain of rabies virus in a Primary Duck embryo fibroblast cell culture in a medium by at least seven passages; and
b) adapting the virus obtained from step (a) in a Primary chick embryo fibroblast cell culture in a medium by at least four passages.

2. A process of adapting a Pitman Moore strain of rabies virus to Primary chick embryo fibroblast cells for the production of rabies vaccine, wherein the process comprises the steps of:
a) adapting the Pitman Moore strain of rabies virus in a Primary Duck embryo fibroblast cell culture in a medium by at least seven passages; and
b) adapting the virus obtained from step (a) in a Primary chick embryo fibroblast cell culture in a medium by at least four passages.

3. The Primary chick embryo fibroblast cell of claim 1, or the process of claim 2, wherein the medium used for adapting the virus in step (a) and step (b) is Production Culture Medium (PCM), which comprises the following ingredients:
| **Ingredient** | **Concentration (Mg/L)** |
|---|---|
| Calcium chloride anhydrous | 175-225 |
| Ferric (III)-Nitrate.9H₂O | 0.02-0.07 |
| Magnesium sulphate anhydrous | 72.7-122.7 |
| Potassium chloride | 375-425 |
| Sodium chloride | 3175-3225 |
| Sodium Dihyrdogen phosphate.H₂O | 107.5-157.5 |
| Sodium hydrogen carbonate | 2925-2975 |
| D-calcium pantothenate | 0.5-5.0 |
| Choline chloride | 0.5-5.0 |
| Folic acid | 0.5-5.0 |
| Myo-Inositol | 0.6-8.6 |
| Nicotinamide | 0.5-5.0 |
| Pyridoxal HCl | 0.5-5.0 |
| Riboflavin | 0.01-0.5 |
| Thiamine HCl | 0.5-5.0 |
| L-arginine HCl | 80-130 |
| L-cystine | 5-30 |
| L-glutamine | 413-463 |
| Glycine | 20-40 |
| L-histidine HCl.H₂O | 17-67 |
| L-isoleucine | 53.5-103.5 |
| L-leucine | 53.5-103.5 |
| L-lysine HCl | 84.25-134.25 |
| L-methionine | 2.5-42.5 |
| L-phenylalanine | 29-74 |
| L-serine | 16-26 |
| L-threonine | 46.5-96.5 |
| L-tryptophan | 10-30 |
| L-tyrosine | 29-79 |
| L-valine | 45-95 |
| D-glucose anhydrous | 2725-2775 |
| Phenol red | 10-30 |
| Sodium pyruvate | 30-80 |

4. The Primary chick embryo fibroblast cell of claim 3, or the process of claim 3, wherein the medium further comprises human serum albumin, hydrolysed gelatin, sodium bicarbonate and suitable antibiotics solution.

5. The Primary chick embryo fibroblast cell of any of claims 1, 3 or 4, or the process of any of claims 2-4, wherein the adaptation is done in PET TC Roller bottles or Multilayered TC flask.

6. The process of claim 2, wherein the process further comprises the step of producing a rabies vaccine from the virus obtained in step (b).

7. Use of the Primary chick embryo fibroblast cell of any of claims 1 or 3-5 in the preparation of a rabies vaccine.

8. A process of preparing a rabies vaccine comprising the steps of -
- obtaining a Master seed by adapting a Pitman Moore strain of rabies virus to Primary chick embryo fibroblast cells in accordance with the process of claim 2;
- preparing the Working seed by a further 1 to 5 passaging of the Master seed in Primary chick embryo fibroblast cells;
- producing the virus from the working seed in PCEC cultures;
- concentrating and purifying the virus;
- inactivating the virus.

9. The process of claim 8, wherein the rabies vaccine is further lyophilized.

10. A rabies vaccine comprising a Pitman Moore strain of rabies virus adapted to Primary chick embryo fibroblast cells obtained according to the process of claim 8 or claim 9.

11. A pharmaceutical composition comprising the rabies vaccine of claim 10 along with suitable excipients.

12. The rabies vaccine of claim 10, for use in vaccinating a mammalian subject.

13. The rabies vaccine for use according to claim 12, wherein the rabies vaccine is to be administered prior to exposure to a pathogenic rabies virus.

14. The rabies vaccine for use according to claim 12, wherein the rabies vaccine is to be administered after the exposure to animal bite which can cause rabies infection.

15. The rabies vaccine for use according to claim 12, wherein the subject is a human.

16. The rabies vaccine for use according to claim 12, wherein the subject is a domestic or wild animal.

17. The rabies vaccine of claim 10, for use in the treatment of rabies in human and animals.

## Patentansprüche

1. Primäre Hühnerembryo-Fibroblastenzelle, die einen Pitman-Moore-Stamm des Tollwutvirus umfasst, das an die primäre Hühnerembryo-Fibroblastenzelle nach einem Verfahren angepasst ist, das folgende Schritte umfasst:
a) Anpassen des Pitman-Moore-Stamms des Tollwutvirus in einer primären Entenembryo-Fibroblastenzellkultur in einem Medium durch mindestens sieben Durchgänge; und
b) Anpassen des Virus, der aus Schritt (a) erhalten wurde, in einer primären Hühnerembryo-Fibroblastenzellkultur in einem Medium durch mindestens vier Durchgänge.

2. Verfahren zum Anpassen eines Pitman-Moore-Stamms des Tollwutvirus an primäre Hühnerembryo-Fibroblastenzellen zur Herstellung eines Tollwutimpfstoffs, wobei das Verfahren folgende Schritte umfasst:
a) Anpassen des Pitman-Moore-Stamms des Tollwutvirus in einer primären Entenembryo-Fibroblastenzellkultur in einem Medium durch mindestens sieben Durchgänge; und
b) Anpassen des Virus, der aus Schritt (a) erhalten wurde, in einer primären Hühnerembryo-Fibroblastenzellkultur in einem Medium durch mindestens vier Durchgänge.

3. Primäre Hühnerembryo-Fibroblastenzelle nach Anspruch 1, oder Verfahren nach Anspruch 2, wobei das Medium, das zum Anpassen des Virus in Schritt (a) und Schritt (b) verwendet wird, ein Produktionskulturmedium (PCM - Production Culture Medium) ist, das folgende Inhaltsstoffe umfasst:
| **Inhaltsstoff** | **Konzentration (mg/l)** |
|---|---|
| Calciumchlorid wasserfrei | 175 - 225 |
| Eisen(III)-Nitrate.9H₂O | 0,02-0,07 |
| Magnesiumsulfat wasserfrei | 72,7 - 122,7 |
| Kaliumchlorid | 375 - 425 |
| Natriumchlorid | 3175 - 3225 |
| Natriumdihyrdogenphosphat.H₂O | 107,5 - 157,5 |
| Natriumhydrogencarbonat | 2925 - 2975 |
| D-Calciumpantothenat | 0,5 - 5,0 |
| Cholinchlorid | 0,5 - 5,0 |
| Folsäure | 0,5 - 5,0 |
| Myo-Inositol | 0,6 - 8,6 |
| Nicotinamid | 0,5 - 5,0 |
| Pyridoxal HCl | 0,5 - 5,0 |
| Riboflavin | 0,01 - 0,5 |
| Thiamin HCl | 0,5 - 5,0 |
| L-Arginin HCl | 80 - 130 |
| L-Cystin | 5 - 30 |
| L-Glutamin | 413 - 463 |
| Glycin | 20 - 40 |
| L-Histidin HCl.H₂O | 17 - 67 |
| L-Isoleucin | 53,5 - 103,5 |
| L-Leucin | 53,5 - 103,5 |
| L-Lysin HCl | 84,25 - 134,25 |
| L-Methionin | 2,5 - 42,5 |
| L-Phenylalanin | 29 - 74 |
| L-Serin | 16 - 26 |
| L-Threonin | 46, 5 - 96,5 |
| L-Tryptophan | 10 - 30 |
| L-Tyrosin | 29 - 79 |
| L-Valin | 45 - 95 |
| D-Glucose wasserfrei | 2725 - 2775 |
| Phenolrot | 10 - 30 |
| Natriumpyruvat | 30 - 80 |

4. Primäre Hühnerembryo-Fibroblastenzelle nach Anspruch 3 oder Verfahren nach Anspruch 3, wobei das Medium ferner Folgendes umfasst: humanes Serumalbumin, hydrolysierte Gelatine, Natriumbicarbonat und eine geeignete Antibiotika-Lösung.

5. Primäre Hühnerembryo-Fibroblastenzelle nach einem der Ansprüche 1, 3 oder 4 oder Verfahren nach einem der Ansprüche 2 - 4, wobei die Anpassung in PET-ZK-Rollerflaschen oder in einem mehrschichtigen ZK-Kolben erfolgt.

6. Verfahren nach Anspruch 2, wobei das Verfahren ferner den Schritt der Herstellung eines Tollwutimpfstoffs aus dem Virus, der in Schritt (b) erhalten wurde, umfasst.

7. Verwendung der primären Hühnerembryo-Fibroblastenzelle nach einem der Ansprüche 1 oder 3 - 5 bei der Herstellung eines Tollwutimpfstoffs.

8. Verfahren zur Herstellung eines Tollwutimpfstoffs das folgende Schritte umfasst:
- Erhalten eines Master-Samens durch Anpassen eines Pitman-Moore-Stamms des Tollwutvirus an primäre Hühnerembryo-Fibroblastenzellen gemäß dem Verfahren von Anspruch 2;
- Herstellen des Arbeitssamens durch weitere 1 bis 5 Durchgänge des Master-Samens in primären Hühnerembryo-Fibroblastenzellen;
- Herstellen des Virus aus dem Arbeitssamen in PCEC-Kulturen;
- Konzentrieren und Reinigen des Virus;
- Inaktivieren des Virus.

9. Verfahren nach Anspruch 8, wobei der Tollwutimpfstoff ferner lyophilisiert wird.

10. Tollwutimpfstoff, der einen Pitman-Moore-Stamm des Tollwutvirus umfasst, der an primäre Hühnerembryo-Fibroblastenzellen angepasst ist, die nach dem Verfahren von Anspruch 8 oder Anspruch 9 erhalten werden.

11. Pharmazeutische Zusammensetzung, die den Tollwutimpfstoff nach Anspruch 10 zusammen mit geeigneten Trägerstoffen umfasst.

12. Tollwutimpfstoff nach Anspruch 10 zur Verwendung bei der Impfung eines Säugers.

13. Tollwutimpfstoff zur Verwendung nach Anspruch 12, wobei der Tollwutimpfstoff vor dem Kontakt mit einem pathogenen Tollwutvirus zu verabreichen ist.

14. Tollwutimpfstoff zur Verwendung nach Anspruch 12, wobei der Tollwutimpfstoff nach einem Tierbiss zu verabreichen ist, der eine Tollwutinfektion verursachen kann.

15. Tollwutimpfstoff zur Verwendung nach Anspruch 12, wobei das Subjekt ein Mensch ist.

16. Tollwutimpfstoff zur Verwendung nach Anspruch 12, wobei das Subjekt ein Haus- oder Wildtier ist.

17. Tollwutimpfstoff nach Anspruch 10 zur Verwendung bei der Behandlung von Tollwut bei Menschen und Tieren.

## Revendications

1. Fibroblaste d'embryon de poulet primaire comprenant une souche Pitman-Moore de virus rabique adaptée au fibroblaste d'embryon de poulet primaire selon un procédé comprenant les étapes qui consistent à :
a) adapter la souche Pitman-Moore de virus rabique dans une culture cellulaire de fibroblastes d'embryons de canards primaires dans un milieu en réalisant au moins sept passages ; et
b) adapter le virus obtenu à l'étape (a) dans une culture cellulaire de fibroblastes d'embryons de poulets primaires dans un milieu en réalisant au moins quatre passages.

2. Procédé d'adaptation d'une souche Pitman-Moore de virus rabique à des fibroblastes d'embryons de poulets primaires pour la production d'un vaccin antirabique, le procédé comprenant les étapes qui consistent à :
a) adapter la souche Pitman-Moore de virus rabique dans une culture cellulaire de fibroblastes d'embryons de canards primaires dans un milieu en réalisant au moins sept passages ; et
b) adapter le virus obtenu à l'étape (a) dans une culture cellulaire de fibroblastes d'embryons de poulets primaires dans un milieu en réalisant au moins quatre passages.

3. Fibroblaste d'embryon de poulet primaire selon la revendication 1, ou procédé selon la revendication 2, le milieu utilisé pour adapter le virus dans l'étape (a) et dans l'étape (b) étant un milieu de culture de production (PCM) qui comprend les ingrédients suivantes :
| **Ingrédient** | **Concentration** (**mg**/**l**) |
|---|---|
| Chlorure de calcium anhydre | 175-225 |
| Nitrate ferrique (III) nonahydraté | 0,02-0,07 |
| Sulfate de magnésium anhydre | 72,7-122,7 |
| Chlorure de potassium | 375-425 |
| Chlorure de sodium | 3 175-3 225 |
| Dihydrogénophosphate de sodium monohydraté | 107,5-157,5 |
| Hydrogénocarbonate de sodium | 2 925-2 975 |
| D-pantothénate de calcium | 0,5-5,0 |
| Chlorure de choline | 0,5-5,0 |
| Acide folique | 0,5-5,0 |
| Myo-inositol | 0,6-8,6 |
| Nicotinamide | 0,5-5,0 |
| Hydrochlorure de pyridoxal | 0,5-5,0 |
| Riboflavine | 0,01-0,5 |
| Hydrochlorure de thiamine | 0,5-5,0 |
| Hydrochlorure de L-arginine | 80-130 |
| L-cystine | 5-30 |
| L-glutamine | 413-463 |
| Glycine | 20-40 |
| Hydrochlorure de L-histidine monohydraté | 17-67 |
| L-isoleucine | 53,5-103,5 |
| L-leucine | 53,5-103,5 |
| Hydrochlorure de L-lysine | 84,25-134,25 |
| L-méthionine | 2,5-42,5 |
| L-phénylalanine | 29-74 |
| L-sérine | 16-26 |
| L-thréonine | 46,5-96,5 |
| L-tryptophane | 10-30 |
| L-tyrosine | 29-79 |
| L-valine | 45-95 |
| D-glucose anhydre | 2 725-2 775 |
| Rouge phénol | 10-30 |
| Pyruvate de sodium | 30-80 |

4. Fibroblaste d'embryon de poulet primaire selon la revendication 3, ou procédé selon la revendication 3, le milieu comprenant en outre de la sérumalbumine humaine, de la gélatine hydrolysée, du bicarbonate de sodium et une solution d'antibiotiques convenant à cette fin.

5. Fibroblaste d'embryon de poulet primaire selon l'une quelconque des revendications 1, 3 ou 4, ou procédé selon l'une quelconque des revendications 2 à 4, l'adaptation étant réalisée dans des flacons roulants de culture cellulaire en PET ou dans un flacon de culture cellulaire multicouche.

6. Procédé selon la revendication 2, le procédé comprenant en outre l'étape de production d'un vaccin antirabique à partir du virus obtenu à l'étape (b).

7. Utilisation du fibroblaste d'embryon de poulet primaire selon l'une quelconque des revendications 1 ou 3 à 5 dans la préparation d'un vaccin antirabique.

8. Procédé de préparation d'un vaccin antirabique, comprenant les étapes qui consistent à :
- obtenir un lot de semence primaire en adaptant une souche Pitman-Moore de virus rabique aux fibroblastes d'embryons de poulets primaires conformément au procédé selon la revendication 2 ;
- préparer le lot de semence de travail en réalisant 1 à 5 passages supplémentaires du lot de semence primaire dans les fibroblastes d'embryons de poulets primaires ;
- produire le virus à partir du lot de semence de travail dans des cultures de cellules purifiées d'embryons de poulets (PCEC) ;
- concentrer et purifier le virus ;
- inactiver le virus.

9. Procédé selon la revendication 8, le vaccin antirabique étant en outre lyophilisé.

10. Vaccin antirabique comprenant une souche Pitman-Moore de virus rabique adaptée à des fibroblastes d'embryons de poulets primaires obtenu conformément au procédé selon la revendication 8 ou la revendication 9.

11. Composition pharmaceutique comprenant le vaccin antirabique selon la revendication 10 conjointement avec des excipients convenant à cette fin.

12. Vaccin antirabique selon la revendication 10, destiné à une utilisation dans la vaccination d'un sujet mammifère.

13. Vaccin antirabique destiné à une utilisation selon la revendication 12, le vaccin antirabique étant destiné à une administration avant une exposition à un virus rabique pathogène.

14. Vaccin antirabique destiné à une utilisation selon la revendication 12, le vaccin antirabique étant destiné à une administration après l'exposition à une morsure d'animal capable de provoquer une infection par la rage.

15. Vaccin antirabique destiné à une utilisation selon la revendication 12, le sujet étant un humain.

16. Vaccin antirabique destiné à une utilisation selon la revendication 12, le sujet étant un animal domestique ou sauvage.

17. Vaccin antirabique selon la revendication 10, destiné à une utilisation dans le traitement de la rage chez les humains et les animaux.
